# EUROPEAN PATENT APPLICATION

(11) **EP 4 841 567 A1**
(43) Date of publication of application: **18.11.2026**
(21) Application number: 26178407.8
(22) Date of filing: 13.05.2026
(51) Int. Cl.: G06T 7/73, A61N 5/10, G06T 7/10

(54) **POSITIONING METHOD AND DEVICE**

(30) Priority: 16.05.2025 CN 202510642006
(71) Applicant: Our United Corporation, Xi'an, Shaanxi 710018 (CN); OUR United [Shenzhen] GAMADAO Technology Co., Ltd., Shenzhen City Guangdong Province 518101 (CN)
(72) Inventor: WANG, Zhongya, Shaanxi, 710018 (CN); ZHANG, Zhongyuan, Shaanxi, 710018 (CN); LI, Jiuliang, Shaanxi, 710018 (CN); LI, Danyu, Shaanxi, 710018 (CN)
(74) Representative: Murgitroyd & Company

(57) **Abstract**

The present disclosure provides a positioning method, comprising: acquiring current body-surface data of a target object; and determining a current position of a target volume of the target object based on the current body-surface data and a pre-established spatial relationship between body-surface data of the target object and the target volume.

## Description

### CROSS-REFERENCE TO RELATED APPLICATIONS

This application claims priority to Chinese Patent Application No. 202510642006.7, filed with the China National Intellectual Property Administration (CNIPA) on May 16, 2025, the entire disclosure of which is incorporated herein by reference.

### TECHNICAL FIELD

The present disclosure relates to the technical field of medical technology, more particularly to treatment-positioning technology, and specifically to a positioning method.

### BACKGROUND

Stereotactic radiosurgery (SRS) is a core treatment modality for tumors and lesions arising in functional regions of a patient, such as deep regions of the nervous system and complex vasculature. By geometrically focusing multiple gamma-ray beams, SRS can achieve high-precision ablation of a target volume with reduced toxic side effects. SRS is applicable to pituitary adenomas, meningiomas, chordomas, and other primary or metastatic tumors of the brain or spine, as well as functional diseases such as trigeminal neuralgia and vascular malformations.

Currently, during an SRS procedure, patient positioning is typically performed manually, for example, during patient setup before treatment and during image guidance in treatment. When operator proficiency is low, the positioning error may be relatively large, thereby affecting treatment efficacy.

### SUMMARY

The present disclosure provides a positioning method performed by a processor. The method comprises acquiring current body-surface data of a target object and determining a current position of a target volume of the target object based on the current body-surface data and a pre-established spatial relationship between body-surface data of the target object and the target volume.

The present disclosure further provides an electronic device including a processor and a memory configured to store instructions executable by the processor, where the processor is configured to execute the instructions to perform the positioning method.

The present disclosure further provides a non-transitory computer-readable storage medium storing a computer program that, when read and executed, causes the positioning method to be performed.

### BRIEF DESCRIPTION OF THE DRAWINGS

The accompanying drawings are provided to facilitate understanding of the present disclosure and do not limit the present disclosure.
FIG. 1 is a schematic diagram of an application environment of a positioning system according to an embodiment of the present disclosure.
FIG. 2 is a schematic flowchart of a positioning method according to an embodiment of the present disclosure.
FIG. 3 is a schematic flowchart of another positioning method according to an embodiment of the present disclosure.
FIG. 4 is a schematic flowchart of another positioning method according to an embodiment of the present disclosure.
FIG. 5 is a schematic flowchart of another positioning method according to an embodiment of the present disclosure.
FIG. 6 is a schematic flowchart of another positioning method according to an embodiment of the present disclosure.
FIG. 7 is a schematic block diagram of an electronic device according to an embodiment of the present disclosure.

### DETAILED DESCRIPTION

The technical solutions in the embodiments of the present disclosure are described below clearly and comprehensively with reference to the accompanying drawings. Obviously, the described embodiments are merely some rather than all embodiments of the present disclosure. All other embodiments that may be derived by a person skilled in the art from the embodiments of the present disclosure without inventive effort shall fall within the protection scope of the present disclosure.

In the description of the present disclosure, the terms "first" "second" and "third" are used for descriptive purposes only. These terms shall not be construed as indicating or implying relative importance or as implicitly specifying the number of the indicated technical features. Thus, technical features defined with "first" "second" and "third" may expressly or implicitly include one or more such technical features. In the description of the present disclosure, "plurality" means two or more, unless otherwise expressly and specifically limited.

In the description of the present disclosure, the term "exemplary" as used herein is intended to mean "serving as an example, instance, or illustration". Any embodiment described as "exemplary" herein shall not necessarily be construed as preferred or advantageous over other embodiments. The following description is provided to enable any person skilled in the art to implement and use the present disclosure. In the following description, details are set forth for explanatory purposes. It should be understood that a person of ordinary skill in the art may recognize that the present disclosure may be implemented without these specific details. In other instances, existing structures and processes are not described in detail to avoid obscuring the description of the present disclosure with unnecessary details. Therefore, the present disclosure is not intended to be limited to the embodiments shown, but is to be accorded the widest scope consistent with the principles and features disclosed herein.

The positioning method provided in the embodiments of the present disclosure is executed by an electronic device. Accordingly, objects processed by the electronic device are represented in the form of data or information. For example, time is essentially represented as time information. In subsequent embodiments, references to size, quantity, position, and the like all refer to corresponding data processed by the electronic device, and details thereof are omitted herein.

During radiotherapy of a patient's tumor, such as SRS-based treatment, the tumor needs to be positioned multiple times, for example, during pre-treatment setup and during in-treatment image guidance. Currently, conventional approaches generally rely on manual operations to perform patient positioning. Low operator proficiency may lead to relatively large positioning errors, thereby affecting treatment efficacy.

To address this technical problem, the embodiments of the present disclosure provide a positioning method and device capable of acquiring current body-surface data of a target object and determining a current position of a target volume of the target object based on the current body-surface data and a pre-established spatial relationship between body-surface data of the target object and the target volume.

More specifically, the spatial relationship may be established in advance between body-surface data of the target object and the target volume of the target object. After the current body-surface data of the target object is acquired, the current position of the target volume may be rapidly and accurately determined based on the spatial relationship, thereby improving positioning accuracy and treatment efficacy.

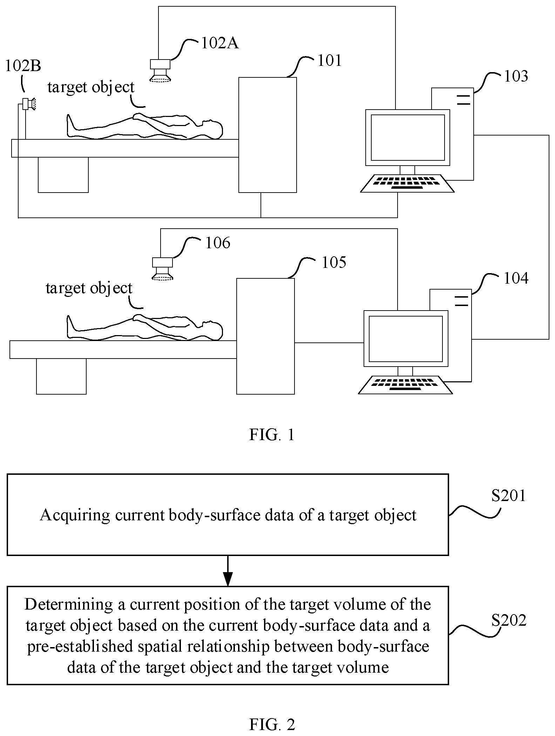

The foregoing positioning method may be applied in a positioning system. FIG. 1 is a schematic diagram of an application environment of a positioning system according to an embodiment of the present disclosure. The positioning system may include radiotherapy equipment 101, one or more body-surface optical acquisition devices, and a first electronic device 103. For example, the body-surface optical acquisition devices may include a first body-surface optical acquisition device 102A and a second body-surface optical acquisition device 102B shown in FIG. 1.

The radiotherapy equipment 101 is a device for performing radiotherapy on a target object. In some embodiments, the radiotherapy equipment 101 may include a gantry, a treatment head, and a treatment couch. Since FIG. 1 is a side view of the radiotherapy equipment 101, the structural schematics of the gantry and the treatment head are not shown in FIG. 1.

The gantry may be a rotating gantry. The treatment head may be disposed on the gantry and configured to emit a therapeutic radiation beam to irradiate a subject. The radiation emitted by the treatment head may include at least one of gamma rays, megavoltage (MV) X-rays, or proton beams. For example, the treatment head may be at least one of a Gamma Knife treatment head for rotational focused radiotherapy, an accelerator treatment head for intensity-modulated conformal radiotherapy, or another radiotherapy treatment head. The treatment couch is configured to support and move the target object.

A body-surface optical acquisition device is configured to acquire body-surface data of a target object, for example, a patient to be treated, an experimental subject, or a phantom, and may be a body-surface optical camera.

In some embodiments, one or more body-surface optical acquisition devices may be provided to acquire current body-surface data of the target object in a pre-treatment setup scenario or an in-treatment irradiation scenario.

For example, the body-surface optical acquisition devices may include the first body-surface optical acquisition device 102A and the second body-surface optical acquisition device 102B shown in FIG. 1.

The first body-surface optical acquisition device 102A may be disposed above the treatment couch of the radiotherapy equipment 101, for example, on the ceiling of the room in which the radiotherapy equipment 101 is located. The first body-surface optical acquisition device 102A may acquire current body-surface data of the target object in a pre-treatment setup scenario.

The second body-surface optical acquisition device 102B may be disposed on the treatment couch of the radiotherapy equipment 101 facing the target object, and may move together with the treatment couch. For example, the second body-surface optical acquisition device 102B may be mounted at the foot end of the treatment couch of the radiotherapy equipment 101 by means of a bracket. The second body-surface optical acquisition device 102B may acquire current body-surface data of the target object in an in-treatment irradiation scenario.

Alternatively, the body-surface optical acquisition device(s) may also be disposed at other positions, so long as current body-surface data of the target object may be acquired in a timely manner during positioning of the target object.

In the embodiments of the present disclosure, the body-surface optical acquisition device(s) are communicatively connected to the first electronic device 103. After acquiring current body-surface data of the target object, the body-surface optical acquisition device(s) may transmit the current body-surface data of the target object to the first electronic device 103, so that the first electronic device 103 performs the subsequent positioning method.

In some embodiments, the first electronic device 103 may be a computer device with a graphical user interface (GUI), and the computer device may include at least one processor, a memory, and at least one application program. For example, the first electronic device 103 may include a positioning-system application program. The processor of the first electronic device 103 executes the positioning-system application program to implement the positioning method provided in the embodiments of the present disclosure.

In the embodiments of the present disclosure, the first electronic device 103 may be implemented as a terminal or as a server with a display. This is not limiting in the embodiments of the present disclosure.

In one embodiment, the terminal may be at least one of a smart phone, a smart watch, a desktop computer, a laptop computer, a virtual reality terminal, an augmented reality terminal, a wireless terminal, or a portable laptop computer.

In one embodiment, the server may be at least one of a standalone physical server, a server cluster or distributed file system composed of multiple physical servers, or a cloud server that provides basic cloud-computing services, such as cloud services, cloud databases, cloud computing, cloud functions, cloud storage, network services, cloud communications, middleware services, domain-name services, security services, content-delivery networks, or big-data or artificial-intelligence platforms. This is not limiting in the embodiments of the present disclosure. In some embodiments, the number of servers may be greater or smaller. The server may further include other functions, to provide more comprehensive and diverse services.

In some embodiments, the first electronic device 103 may run a computer system. The computer system includes a processor configured to implement the positioning method provided by the embodiments of the present disclosure.

In one embodiment, the first electronic device 103 may also be communicatively coupled to the radiotherapy equipment 101, so that the radiotherapy equipment 101 may adjust the position of the target object on the radiotherapy equipment according to the positioning result for the target volume of the target object determined by the first electronic device 103, namely, the current position of the target volume.

In some embodiments, the positioning method provided by the present disclosure may further include establishing the spatial relationship between body-surface data of the target object and the target volume. In this case, the positioning system may further include a simulation system. The simulation system may include a second electronic device 104 and a simulated locator 105.

In some embodiments, the simulated locator 105 may be at least one of a computed tomography (CT) device, an emission computed tomography (ECT) device, a magnetic resonance imaging (MRI) device, a positron emission tomography (PET) device, or an ultrasound examination device.

In the embodiments of the present disclosure, the simulated locator 105 is communicatively connected to the second electronic device 104. After acquiring a positioning image of the target object, the simulated locator 105 may transmit the positioning image of the target object to the second electronic device 104. The second electronic device 104 establishes a spatial relationship according to body-surface data in the positioning image and a position of the target volume, and transmits the spatial relationship to the first electronic device 103.

In one embodiment, after acquiring the positioning image of the target object, the simulated locator 105 may also directly transmit the positioning image of the target object to the first electronic device 103, so that the first electronic device 103 establishes the spatial relationship according to body-surface data in the positioning image and the position of the target volume.

In one embodiment, the simulation system may further include a body-surface optical acquisition device 106. The body-surface optical acquisition device 106 may acquire body-surface data of the target object at the same time that the simulated locator 105 acquires the positioning image. Subsequently, the body-surface optical acquisition device 106 may transmit the body-surface data of the target object to the first electronic device 103 or the second electronic device 104, so that the first electronic device 103 or the second electronic device 104 establishes the spatial relationship.

In one embodiment, one or more body-surface optical acquisition devices 106 may be provided and may be disposed on the ceiling of the room in which the simulated locator 105 is located, or on a support device of the simulated locator 105, for example, a simulation couch. This is merely illustrative and not limiting in the embodiments of the present disclosure. FIG. 1 illustrates, by way of example, a case in which the body-surface optical acquisition device 106 is disposed on the ceiling of the room in which the simulated locator 105 is located.

In one embodiment, the physical structure of the second electronic device 104 may refer to the description of the physical structure of the first electronic device 103, and the details will not be repeated herein.

In one embodiment, the first electronic device 103 and the second electronic device 104 may be integrated into the same device, that is, the first electronic device 103 and the second electronic device 104 may be the same electronic device. This is not limiting in the embodiments of the present disclosure.

In one embodiment, the radiotherapy equipment 101 may further include an image-guidance device configured to acquire a guidance image of the target object. Exemplarily, the image-guidance device may include an X-ray tube and a detector arranged opposite each other on the gantry. The detector may receive an imaging beam emitted by the X-ray tube and passing through the target object, and may generate a projection image. The first electronic device 103 may acquire the guidance image of the target object and may verify accuracy of an error-evaluation result based on the guidance image of the target object and a positioning reference image. The error-evaluation result may be obtained by evaluating a positional offset, and the positional offset may be an offset between the current body-surface data and body-surface data in the spatial relationship.

The positioning method provided in the embodiments of the present disclosure may be applied to the first electronic device 103 in FIG. 1. As shown in FIG. 2, the positioning method includes steps S201 and S202.

S201. Acquiring current body-surface data of a target object.

In one embodiment, the current body-surface data may be current body-surface data of the target object in a current scenario. The current scenario may be a pre-treatment setup-adjustment scenario or an in-treatment irradiation scenario for the target object.

Specifically, in a scenario of pre-treatment setup-adjustment or irradiation during treatment of the target object, current body-surface data of the target object may generally be acquired by a body-surface optical acquisition device, for example, body-surface optical acquisition device 102A or 102B in FIG. 1.

S202. Determining a current position of the target volume of the target object based on the current body-surface data and a pre-established spatial relationship between body-surface data of the target object and the target volume.

As shown in step S202 of FIG. 2, the first electronic device may determine the current position of the target volume based on the current body-surface data and the pre-established spatial relationship between the body-surface data of the target object and the target volume. In this manner, the target volume may be rapidly and accurately located, thereby improving positioning accuracy and treatment efficacy.

In one embodiment, the pre-established spatial relationship between body-surface data of the target object and the target volume of the target object may be stored in a memory of the first electronic device or in a storage server communicatively connected to the first electronic device, for example, the second electronic device 104 in FIG. 1. After acquiring the current body-surface data, the first electronic device may read the pre-established spatial relationship from the memory or the storage server so as to determine the current position of the target volume of the target object based on the spatial relationship and the current body-surface data.

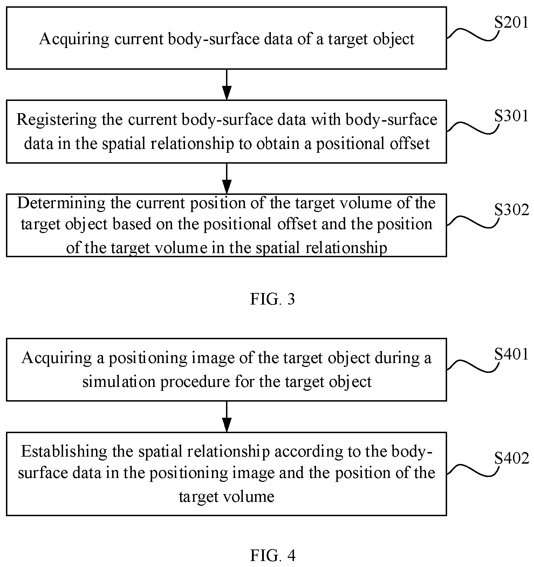

In some embodiments, with reference to FIG. 2 and as shown in FIG. 3, the method by which the first electronic device determines the current position of the target volume of the target object based on the current body-surface data and the pre-established spatial relationship between body-surface data of the target object and the target volume specifically includes steps S301 to S302.

S301. Registering the current body-surface data with body-surface data in the spatial relationship to obtain a positional offset.

Specifically, because the current body-surface data is body-surface data acquired at a current time and the body-surface data in the spatial relationship is pre-established body-surface data, temporal or spatial differences may exist between the two sets of body-surface data. Therefore, the first electronic device may register the current body-surface data with the body-surface data in the spatial relationship to obtain a positional offset.

In some embodiments, the method by which the first electronic device registers the current body-surface data with the body-surface data in the spatial relationship to obtain the positional offset specifically includes:

Performing initial registration on the current body-surface data and the body-surface data in the spatial relationship to obtain an initial registration result; and adjusting the initial registration result based on a pre-trained deep-learning model to obtain the positional offset.

Specifically, registration between the current body-surface data and the body-surface data in the spatial relationship may be performed in the following Coarse-registration procedure and Fine-registration procedure.

Coarse-registration procedure: registration is performed according to a hierarchical registration strategy, namely, coarse registration. More specifically, coarse registration is first performed on the current body-surface data and the body-surface data in the spatial relationship using a registration algorithm having a relatively high registration speed, thereby quickly obtaining the initial registration result.

Fine-registration procedure: the initial registration result is adjusted, namely, fine registration is performed, using a deep-learning model having relatively high registration accuracy, so as to obtain the positional offset.

Accordingly, a multimodal registration engine may be constructed through the Coarse-registration procedure and the Fine-registration procedure to register the body-surface data.

In one embodiment, the Coarse-registration procedure may use an Iterative Closest Point (ICP) algorithm. The algorithm may complete initial registration within 2 s. Other fast registration algorithms may also be used in the coarse-registration procedure. This is not limiting in the embodiments of the present disclosure.

In one embodiment, in the Fine-registration procedure, the deep-learning model may estimate a deformation field between the two sets of body-surface data in the initial registration result, thereby obtaining an accurate registration result. An average registration error of the registration result may be less than or equal to 0.5mm.

In one embodiment, the deep-learning model used in the Fine-registration procedure may be obtained through training based on a large number of training samples. In addition, the deep-learning model may be updated by acquiring new training samples in real time, that is, an incremental-learning mechanism may be introduced. For example, the new training samples may include data from 100 new patients. Experimental results show that, for special body types, the registration success rate of the deep-learning model may be increased to above 90%, where a special body type refers to a body type with a body mass index (BMI) greater than 30. For ordinary body types, the registration success rate may be higher.

In some embodiments, when registering the current body-surface data with the body-surface data in the spatial relationship, the first electronic device may use all or part of the current body-surface data, such as the landmarks described below, and all or part of the body-surface data in the spatial relationship. In step S201, the first electronic device registers the current body-surface data with body-surface data in the spatial relationship to obtain a positional offset. The registration may be performed in any of the following four scenarios.

Registration scenario 1: the first electronic device registers all current body-surface data against all body-surface data in the spatial relationship to obtain the positional offset.

To obtain an accurate registration result, i.e., the positional offset, the first electronic device may register all current body-surface data against all body-surface data in the spatial relationship.

Registration scenario 2: the first electronic device registers landmarks extracted from the current body-surface data against all body-surface data in the spatial relationship to obtain the positional offset.

Since the target object typically includes landmarks that support fast registration, the first electronic device may extract those landmarks from the current body-surface data and register the landmarks against all body-surface data in the spatial relationship to obtain the positional offset.

In some embodiments, the landmarks may include at least one of a zygomatic bone, a nasal tip, a brow ridge, an anterior superior iliac spine, or a sternal angle.

Registration scenario 3: the first electronic device registers all current body-surface data against landmarks corresponding to the body-surface data in the spatial relationship to obtain the positional offset.

In Registration scenario 3, the first electronic device may extract landmarks for fast registration from the body-surface data in the spatial relationship, and may register all current body-surface data against those landmarks to obtain the positional offset.

Registration scenario 4: the first electronic device registers landmarks extracted from the current body-surface data against landmarks corresponding to the body-surface data in the spatial relationship to obtain the positional offset.

In Registration scenario 4, the first electronic device may extract landmarks for fast registration from the current body-surface data and from the body-surface data in the spatial relationship, respectively. The first electronic device may then register the landmarks extracted from the current body-surface data against the landmarks corresponding to the body-surface data in the spatial relationship to obtain the positional offset.

In Registration scenarios 2-4, the first electronic device uses landmarks to perform fast registration. Accordingly, before the positional offset is obtained, the method may further include extracting landmarks by the first electronic device based on a surface feature extraction model.

The landmarks include landmarks corresponding to the current body-surface data, or landmarks corresponding to the body-surface data in the spatial relationship.

In one embodiment, the surface feature extraction model may be a U-Net++ model. The body-surface data in the spatial relationship may be derived from a positioning image, for example, a CT image acquired by a computed tomography (CT) image-acquisition device.

The U-Net++ model has a nested skip-connection structure, thereby enabling efficient fusion of multi-scale anatomical features in a CT image, such as large-scale skeletal contours and subtle body-surface data, so as to accurately capture landmarks such as the zygomatic bone and the nasal tip. In addition, the deep-supervision mechanism provided in the U-Net++ model can significantly improve segmentation accuracy at low-contrast soft-tissue boundaries, so as to accommodate a complex CT-noise environment and thereby accurately extract landmarks from the body-surface data. Further, extensive experimental verification, for example, verification in medical-imaging tasks involving the liver, lungs, and other organs, shows that the U-Net++ model can balance robustness to individual differences and noise with computational efficiency. Accordingly, landmarks in body-surface data can be extracted quickly and accurately by means of the U-Net++ model.

In some embodiments, because the amount of body-surface data is large while landmarks are typically located only at certain particular positions, the first electronic device may first determine a position region in corresponding body-surface data to which a landmark belongs, and then extract the landmark based on the surface feature extraction model. In this case, the method by which the first electronic device extracts the landmark based on the surface feature extraction model specifically includes:

Determining, based on a landmark template library preconstructed according to prior knowledge, a position region in corresponding body-surface data to which the landmarks belong; and extracting the landmarks from the position region in the corresponding body-surface data based on the surface feature extraction model.

The landmark template library includes positions of landmarks in different types of body-surface data.

Specifically, the prior knowledge may include a large number of different types of body-surface data. The landmark template library may be constructed by acquiring positions of landmarks in these different types of body-surface data. Subsequently, the first electronic device may first determine, according to the landmark template library, a position region in corresponding body-surface data to which the landmarks belong. For example, a nasal-tip landmark is typically located in a middle facial region, and the first electronic device may then extract the landmark from the corresponding position region based on the surface feature extraction model.

S302. Determining the current position of the target volume of the target object based on the positional offset and the position of the target volume in the spatial relationship.

Specifically, after the positional offset is obtained, since the pre-established spatial relationship typically does not change, the first electronic device may determine the current position of the target volume of the target object based on the positional offset and the position of the target volume in the spatial relationship.

In some embodiments, the method for establishing the spatial relationship between the body-surface data and the target volume may be applied to the second electronic device 104 in FIG. 1. As shown in FIG. 4, the spatial relationship may be established in the following manner:

S401. Acquiring a positioning image of the target object during a simulation procedure for the target object.

Specifically, the simulation procedure refers to a procedure in which simulation positioning is performed on the target volume of the target object.

In some embodiments, the positioning image may include at least one of a CT image, an ECT image, an MRI image, a PET image, or an ultrasound image.

When the positioning image is a CT image, the second electronic device may generate body-surface data of the target object, for example, RT Structure (RTSTRUCT) data, based on the CT image.

S402. Establishing the spatial relationship according to the body-surface data in the positioning image and the position of the target volume.

Specifically, since the accurate position of the target volume of the target object needs to be acquired during the simulation procedure, after the positioning image is acquired, the second electronic device may extract the position of the target volume from the positioning image. Meanwhile, the second electronic device may also extract body-surface data of the target object based on the positioning image.

The second electronic device may then correlate the body-surface data in the positioning image with the position of the target volume, so as to establish the spatial relationship.

In one embodiment, the second electronic device may also directly acquire body-surface data of the target object from a body-surface point-cloud image.

In one embodiment, after acquiring the body-surface data of the target object, the second electronic device may transform a coordinate system of the body-surface data in the body-surface point-cloud image into a coordinate system of the position of the target volume, namely, the coordinate system of the CT device, so as to subsequently establish the spatial relationship between body-surface data of the target object and the target volume in a common coordinate system.

In some embodiments, when the target object is a living subject, the body-surface data and the target volume of the target object may vary with respiration of the target object. Since respiration of the target object generally includes different phases, when establishing the spatial relationship, the second electronic device may further acquire a respiratory phase of the target object, and may then establish a relationship between body-surface data and the position of the target volume for each respiratory phase. In other words, the spatial relationship may be a relationship between body-surface data and a position of the target volume for each respiratory phase.

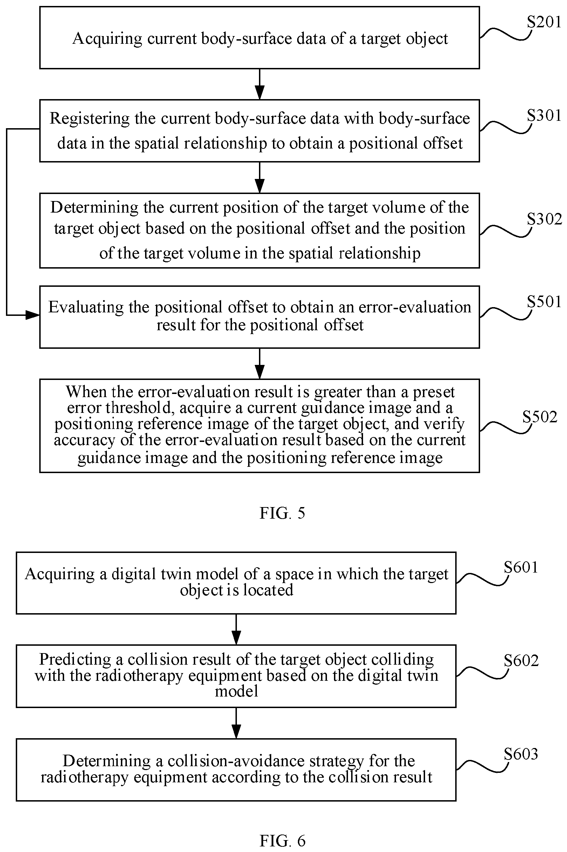

In some embodiments, after registering the current body-surface data with the body-surface data in the spatial relationship to obtain the positional offset, the first electronic device may further evaluate the positional offset. In this case, with reference to FIG. 3 and as shown in FIG. 5, the method provided in the embodiments of the present disclosure may further include steps S501 to S502.

S501. Evaluating the positional offset to obtain an error-evaluation result for the positional offset.

In one embodiment, the first electronic device may evaluate the positional offset using a distance algorithm, for example, a Hausdorff algorithm, so as to obtain the error-evaluation result.

In one embodiment, the first electronic device may evaluate the positional offset using a similarity algorithm, for example, a Dice coefficient algorithm, so as to obtain the error-evaluation result.

In one embodiment, the first electronic device may evaluate the positional offset using both a distance algorithm and a similarity algorithm, so as to obtain the error-evaluation result.

S502. When the error-evaluation result is greater than a preset error threshold, acquire a current guidance image and a positioning reference image of the target object, and verify accuracy of the error-evaluation result based on the current guidance image and the positioning reference image.

In one embodiment, the current guidance image and the positioning reference image may be two-dimensional images. For example, the current guidance image may be an X-ray projection image corresponding to a particular imaging angle, and the positioning reference image may be a Digitally Reconstructed Radiograph (DRR) image generated from the positioning image at the same imaging angle, specifically, a two-dimensional digital projection image generated by digitally reconstructing the positioning image at that imaging angle.

In one embodiment, the current guidance image and the positioning reference image may also be three-dimensional images. For example, the current guidance image may be a CBCT image obtained by three-dimensional reconstruction based on X-ray projection images acquired at different imaging angles, and the positioning reference image may be the positioning image.

In one embodiment, the positioning image may include at least one of a CT image, an ECT image, an MRI image, a PET image, or an ultrasound image.

In some embodiments, as shown in FIG. 6, the method provided in the embodiments of the present disclosure may further include steps S601 to S603.

S601. Acquiring a digital twin model of a space in which the target object is located.

The digital twin model includes equipment parameters of radiotherapy equipment, position and posture parameters of the target object, and a relative position between the target object and the radiotherapy equipment.

In one embodiment, to accurately predict whether the target object will collide with the radiotherapy equipment, a digital twin model of the space in which the target object is located may be established. The digital twin model may be established using a general modeling technique, and the first electronic device may acquire the digital twin model of that space.

S602. Predicting a collision result of the target object colliding with the radiotherapy equipment based on the digital twin model.

In one embodiment, after acquiring the digital twin model of the space in which the target object is located, the first electronic device may construct a bounding volume for the target object based on a bounding volume hierarchy (BVH). The first electronic device may then use a GPU-accelerated ray-casting method, which detects potential collisions ten times per second, to perform collision detection on the BVH constructed for the target object, thereby predicting a collision result of the target object with the radiotherapy equipment.

For example, the collision result predicted in step S602 may be classified as a first result, a second result, or a third result.

The first result may correspond to a collision result having a safety distance greater than 5 mm. The second result may correspond to a collision result having a safety distance between 2 mm and 5 mm. The third result may correspond to a collision result having a safety distance less than 2 mm.

S603. Determining a collision-avoidance strategy for the radiotherapy equipment according to the collision result.

When the collision result is the first result, i.e., when the safety distance exceeds 5 mm, the collision-avoidance strategy may include issuing a yellow warning and outputting collision prompt information.

When the collision result is the second result, i.e., when the safety distance is between 2 mm and 5 mm, the collision-avoidance strategy may include issuing an orange warning and automatically adjusting a treatment path of the radiotherapy equipment.

When the collision result is the third result, i.e., when the safety distance is less than 2 mm, the collision-avoidance strategy may include issuing a red warning and performing an emergency stop on the radiotherapy equipment.

The foregoing description primarily addresses the embodiments of the present disclosure from a method perspective. To implement the corresponding functions, the first electronic device may include corresponding hardware structures and/or software modules configured to perform those functions.

According to embodiments of the present disclosure, an electronic device is further provided, including at least one processor and a memory communicatively coupled to the at least one processor. The memory stores instructions executable by the at least one processor, and the instructions, when executed by the at least one processor, cause the at least one processor to perform the positioning method of the present disclosure.

According to embodiments of the present disclosure, a non-transitory computer-readable storage medium is further provided. The non-transitory computer-readable storage medium stores computer instructions that, when executed, cause an electronic device to perform the positioning method provided by the present disclosure.

According to embodiments of the present disclosure, a computer program product is further provided. The computer program product includes a computer program that, when executed by a processor, implements the positioning method of the present disclosure.

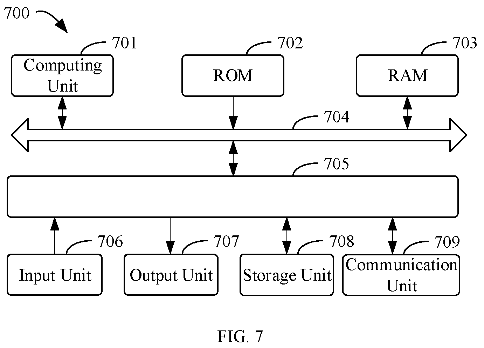

FIG. 7 shows a schematic block diagram of an example electronic device 700 that may be used to implement embodiments of the present disclosure. The electronic device is intended to represent various forms of digital computers, such as workstations, servers, mainframe computers, and other suitable computers. The components shown herein, their connections and relationships, and their functions are merely examples and are not intended to limit implementation of the present disclosure described and/or claimed herein. In some embodiments, the electronic device may be the first electronic device 103 or the second electronic device 104 shown in FIG. 1 .

As shown in FIG. 7, the electronic device 700 includes a computing unit 701, which may perform various appropriate actions and processes according to a computer program stored in a read-only memory (ROM) 702 or a computer program loaded from a storage unit 708 into a random access memory (RAM) 703. Various programs and data for operation of the electronic device 700 may also be stored in the RAM 703. The computing unit 701, the ROM 702, and the RAM 703 are connected to one another through a bus 704. An input/output (I/O) interface 705 is also connected to the bus 704.

A plurality of components in the electronic device 700 are connected to the I/O interface 705, including: an input unit 706, such as a keyboard or a mouse; an output unit 707, such as various types of displays or speakers; a storage unit 708, such as a magnetic disk or an optical disk; and a communication unit 709, such as a network card, a modem, or a wireless communication transceiver. The communication unit 709 enables the electronic device 700 to exchange information/data with other devices via at least one computer network, such as the Internet, or various telecommunication networks.

The computing unit 701 may include one or more general-purpose and/or special-purpose processing components with processing and computing capabilities. Examples of the computing unit 701 include, but are not limited to, a central processing unit (CPU), a graphics processing unit (GPU), various dedicated artificial-intelligence (AI) computing chips, various computing units running machine-learning model algorithms, a digital signal processor (DSP), and any suitable processor, controller, or microcontroller. The computing unit 701 performs the various methods and processes described herein, such as the positioning method described in connection with FIGS. 2-6.

For example, in one embodiment, the positioning method may be implemented as a computer software program tangibly embodied in a machine-readable medium, such as the storage unit 708. In one embodiment, part or all of the computer program may be loaded onto and/or installed on the electronic device 700 via at least one of the ROM 702 or the communication unit 709. When the computer program is loaded into the RAM 703 and executed by the computing unit 701, one or more steps of the positioning method described herein may be performed. Alternatively, in other embodiments, the computing unit 701 may be configured to perform the positioning method in another suitable manner, for example, by means of firmware.

It should be understood that the steps in the various process forms shown in FIGS. 2-6 may be reordered, added, or deleted. For example, the steps recited in the present disclosure may be executed in parallel, sequentially, or in another order, without limitation, so long as the desired results of the technical solutions of the present disclosure are achieved.

The specific implementations described herein do not limit the protection scope of the present disclosure. Those skilled in the art will appreciate that various modifications, combinations, sub-combinations, and substitutions may be made according to design requirements and other factors. Any modification, equivalent replacement, or improvement made within the spirit and principle of the present disclosure shall fall within the protection scope of the present disclosure.

## Claims

1. A positioning method performed by a processor, comprising:
acquiring current body-surface data of a target object; and
determining a current position of a target volume of the target object based on the current body-surface data and a pre-established spatial relationship between body-surface data of the target object and the target volume.

2. The positioning method of claim 1, wherein determining the current position of the target volume of the target object based on the current body-surface data and the pre-established spatial relationship between body-surface data of the target object and the target volume comprises:
registering the current body-surface data with body-surface data in the spatial relationship to obtain a positional offset; and
determining the current position of the target volume of the target object based on the positional offset and a position of the target volume in the spatial relationship.

3. The positioning method of claim 2, wherein registering the current body-surface data with the body-surface data in the spatial relationship to obtain the positional offset comprises at least one of:
registering all of the current body-surface data with all of the body-surface data in the spatial relationship to obtain the positional offset;
registering landmarks corresponding to the current body-surface data with all of the body-surface data in the spatial relationship to obtain the positional offset;
registering all of the current body-surface data with landmarks corresponding to the body-surface data in the spatial relationship to obtain the positional offset; or
registering the landmarks corresponding to the current body-surface data with landmarks corresponding to the body-surface data in the spatial relationship to obtain the positional offset.

4. The positioning method of claim 2, wherein registering the current body-surface data with the body-surface data in the spatial relationship to obtain the positional offset comprises:
performing initial registration on the current body-surface data and the body-surface data in the spatial relationship to obtain an initial registration result; and
adjusting the initial registration result based on a pre-trained deep-learning model to obtain the positional offset.

5. The positioning method of claim 3, further comprising, before registering the current body-surface data with the body-surface data in the spatial relationship to obtain the positional offset:
extracting landmarks based on a surface feature extraction model,
wherein the landmarks are landmarks corresponding to the current body-surface data or corresponding to the body-surface data in the spatial relationship.

6. The positioning method of claim 5, wherein extracting the landmarks based on the surface feature extraction model comprises:
determining, based on a landmark template library pre-constructed according to prior knowledge, a position region in corresponding body-surface data to which the landmarks belong, wherein the landmark template library includes positions of landmarks in different types of body-surface data; and
extracting the landmarks from the position region in the corresponding body-surface data based on the surface feature extraction model.

7. The positioning method of claim 3, wherein the landmarks include at least one of a zygomatic bone, a nasal tip, a brow ridge, an anterior superior iliac spine, or a sternal angle.

8. The positioning method of claim 2, further comprising, after registering the current body-surface data with the body-surface data in the spatial relationship to obtain the positional offset:
evaluating the positional offset to obtain an error-evaluation result for the positional offset; and
when the error-evaluation result is greater than a preset error, acquiring a current guidance image and a positioning reference image of the target object, and verifying accuracy of the error-evaluation result based on the current guidance image and the positioning reference image.

9. The positioning method of claim 1, further comprising:
acquiring a digital twin model of a space in which the target object is located, wherein the digital twin model includes equipment parameters of radiotherapy equipment, position and posture parameters of the target object, and a relative position between the target object and the radiotherapy equipment;
predicting a collision result of the target object colliding with the radiotherapy equipment based on the digital twin model; and
determining a collision-avoidance strategy for the radiotherapy equipment according to the collision result.

10. The positioning method of claim 1, wherein the spatial relationship is a relationship between body-surface data and a position of the target volume for each respiratory phase.

11. The positioning method of claim 1, wherein the spatial relationship is established by:
acquiring a positioning image of the target object during a simulation procedure for the target object; and
establishing the spatial relationship according to body-surface data in the positioning image and a position of the target volume.

12. The positioning method of claim 11, wherein the positioning image includes at least one of a computed tomography image, an emission computed tomography image, a magnetic resonance imaging image, a positron emission tomography image, or an ultrasound image.

13. An electronic device, comprising:
a processor; and
a memory configured to store instructions executable by the processor;
wherein the processor is configured to execute the instructions to perform operations comprising:
acquiring current body-surface data of a target object; and
determining a current position of a target volume of the target object based on the current body-surface data and a pre-established spatial relationship between body-surface data of the target object and the target volume.
